# EUROPEAN PATENT APPLICATION

(11) **EP 0 878 193 A1**
(43) Date of publication of application: **18.11.1998**
(21) Application number: 97901791.0
(22) Date of filing: 30.01.1997
(51) Int. Cl.: A61K 31/19

(54) **REMEDIES FOR HERPESVIRUS INFECTION CAUSED BY HERPESVIRUS AND PREVENTIVES FOR RECURRENCE OF THE INFECTION BOTH CONTAINING TRITERPENE DERIVATIVES AS THE ACTIVE INGREDIENT**

(30) Priority: 02.02.1996 JP 40685/96
(71) Applicant: Showa Shell Sekiyu K.K., Tokyo 135 (JP)
(72) Inventor: HOZUMI, Toyoharu, Showa Shell Sekiyu K.K., Tokyo 135 (JP); OOYAMA, Haruo, Yokohama-shi, Kanagawa 241 (JP); SHIRAKI, Kimiyasu, Toyama-shi, Toyama 930 (JP); KUROKAWA, Masahiko, Imizu-gun, Toyama 939-03 (JP); NAMBA, Tsuneo, Toyama-shi, Toyama 930 (JP); KADOTA, Shigetoshi, Toyama-shi, Toyama 930 (JP)
(74) Representative: HOFFMANN - EITLE
(86) International application number: JP9700224
(87) International publication number: WO9727849

(57) **Abstract**

This invention provides novel agents for the treatment of herpesvirus infections and for the prevention of recurrence of herpesvirus infections, which are derived from a crude drug and can be administered for a prolonged period of time without causing the problems involved in the prior art anti-herpesvirus agents comprised of nucleic acid derivatives, namely generation of drug-resistant strains and induction of side effects. Particularly, it provides an agent for use in the treatment of herpesvirus infections, which comprises 3-oxo-olean-18-en-28-oic acid represented by the following formula (I): 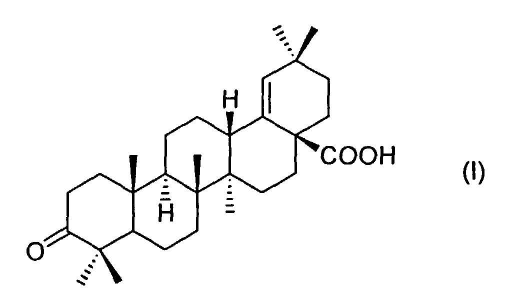 as an active ingredient, and an agent for the prevention of recurrence of herpesvirus infections, which comprises 3-oxo-olean-18-en-28-oic acid represented by the above formula (I) as an active ingredient.

## Description

### TECHNICAL FIELD

This invention relates to an agent for the treatment of herpesvirus infections and an agent for the prevention of recurrence of herpesvirus infections.

### BACKGROUND ART

Japanese galls are produced from bag-like insect galls formed on young buds and leaves of *Rhus javanica L.* belonging to the family *Anacardiaceae* by the stimulation of an aphid *Malaphis chinensis Bell* parasitized thereon, by soaking the insect galls in hot water for a while and then drying them. It is known that tannin occupies 70 to 80% of their components so that they are used as an astringent-antidiarrheal drug, an antitussive, a hemostatic and a sweat reducing agent, and the inventors of the present invention have found for the first time that Japanese galls were effective as a herpesvirus treating agent and applied a patent as JP-A-6-25003 based on the finding.

On the other hand, all of the drugs so far used as antiviral agents are nucleic acid derivatives, excluding interferon. Particularly, Aciclovir which is a trade name and manufactured by The Wellcome Foundation Ltd., namely 9-(2-hydroxy-ethoxymethyl)guanine represented by the following formula (II): 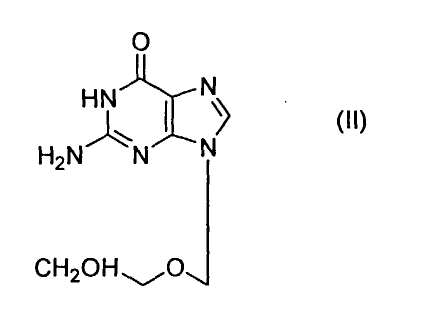 is broadly used as a herpes treating agent.

By the way, since the above-described hot water extract of Japanese galls contains various substances, it was not clear about which one of these substances is acting as the therapeutic agent of herpesvirus infections.

The most significant characteristics common in viruses of the family *Herpesviridae* are latent infection and recurrence (palindromic crisis). Surprisingly, such a recurrence occurs even in the presence of antibodies.

It is known that recurrence of herpes simplex virus after initial infection of the body surface occurs through a root of latent infection into sensory nerve-nervous system-formation of foci in sensory nerve-nerve controlling region.

In the case of human, herpes simplex virus type 1 after causing gingivitis by the initial infection passes through the trigeminal nerve and reaches the trigeminal ganglion where it becomes latent. Caused by a certain factor, the virus in latent condition starts to propagate in the ganglion and again passes through the trigeminal nerve to propagate in cells in the body surface, thereby causing labial herpes. The factors which induce its recurrence in the case of human are fever, prolonged exposure to sunlight (ultraviolet rays), stress, fatigue, menstruation and the like, and frequent recurrence is becoming a serious problem.

On the other hand, herpes simplex virus type 2 causes vulvovaginitis. The herpes simplex virus type 2 ascends a synsacrum-side sensory nerve into the synsacrum dorsal root ganglion where it becomes latent, but starts to propagate therein triggered by a certain inducing factor and again descends into the sensory nerve to cause genital herpes as a palindromic crisis. Similar to the case of herpes simplex virus type 1, the factors which induce its recurrence in the case of human are fever, ultraviolet rays, stress, fatigue, menstruation and the like.

Two problems occur when recurrence of such a genital herpes is induced accompanied by pregnancy. One of them is a possibility of generating malformation in a fetus or of causing abortion, premature delivery or stillbirth, due to infection of the fetus in the womb with the virus, and the other one is that a fetus is infected in the birth canal at the time of delivery and becomes an infection source of neonatal herpes.

Since the number of cases of the birth of deformed babies caused by the fetal infection is extremely small, its influences are not clear, but neonatal herpes caused by birth canal infection is a disease having markedly high fatality rate and there is no sure method that can treat the disease after its onset, so that prevention of the infection becomes important.

The mechanism of latent infection and recurrence is still unclear on many points, so that there are many problems to be solved such as the mechanism of palindromic crisis in the presence of neutralizing antibodies, the means of curing the disease after its recurrence by inhibiting propagation of the virus, the induction mechanism of palindromic crisis and the conditions of the virus in latent stage. Since many of these problems still remain unsolved, frequent recurrence is a serious problem in relation to pregnancy.

In order to prevent palindromic crisis of such herpes viruses, it is necessary to inhibit propagation of the viruses in their latent stage. For that purpose, it is necessary to administer a drug which can inhibit propagation of these viruses, prior to their activation.

The use of the above-described 9-(2-hydroxyethoxymethyl)guanine as an agent for the prevention of recurrence of herpes simplex virus type 2 has been reported for example by S.E. Straus *et al.* (*N. Engl. J. Med*., Vol.310, pp.1545 - 1550, 1984), J.M. Douglas *et al.* (*N. Engl. J. Med.*, Vol.310, pp.1551 - 1556, 1984) and L.G. Kaplowitz et al. (*JAMA*, Vol.265, pp.747 - 751, 1991).

However, when the above-described 9-(2-hydroxyethoxymethyl)guanine is administered for a prolonged period of time, it causes problems such as generation of drug-resistant strains and induction of side effects, thereby making discontinuation of the administration unavoidable. Because of this, great concern has been directed toward the development of a new recurrence preventing agent which is free from these problems.

An object of the present invention is to provide novel agents for the treatment of herpesvirus infections and prevention of recurrence of the infections, which are derived from a crude drug and can be administered for a prolonged period of time without causing the problems involved in the previous anti-herpesvirus agents comprising nucleic acid derivatives, namely generation of drug-resistant strains and induction of side effects.

### DISCLOSURE OF THE INVENTION

The inventors of the present invention have found that moronic acid (namely 3-oxo-olean-18-en-28-oic acid), a compound extracted with hot water from Japanese galls (*Rhus javanica L*.), has an effect to treat herpes and an effect to prevent recurrence of herpesvirus without causing generation of drug-resistant strains and induction of side effects, thereby resulting in the accomplishment of the present invention.

A hot water extract of *Rhus javanica L.* (the reflux extract of Example 1) showed an effective concentration (EC₅₀) of 56.7 ± 6.0 µg/mℓ which inhibits 50% of viral growth. In addition, when the hot water extract was fractionated by ethyl acetate extraction with varied pH values, a fraction of pH 10 showed the most strong antiviral activity and strong cytotoxicity, with its EC₅₀ value of 7.4 ± 2.0 µg/mℓ. The EC₅₀ value of a pH 3.6 fraction was 27.5 µg/mℓ, and the EC₅₀ value of a pH 7.0 fraction was 16.5 µg/mℓ. Evaluation of the antiviral activity of these samples was carried out by a plaque formation test.

Accordingly, the present invention relates to an agent for treating herpesvirus infections, which comprises 3-oxo-olean-18-en-28-oic acid represented by the following formula (I): 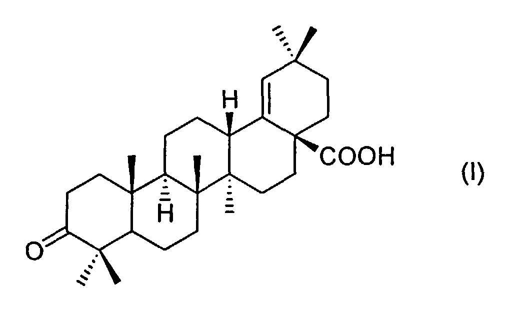 as an active ingredient.

Also, the present invention relates to an agent for preventing recurrence of herpesvirus infections, which comprises 3-oxo-olean-18-en-28-oic acid represented by the following formula (I): 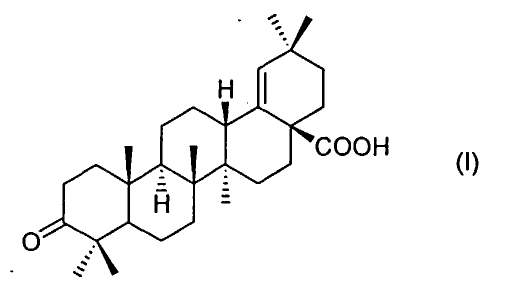 as an active ingredient.

### BRIEF DESCRIPTION OF THE DRAWINGS

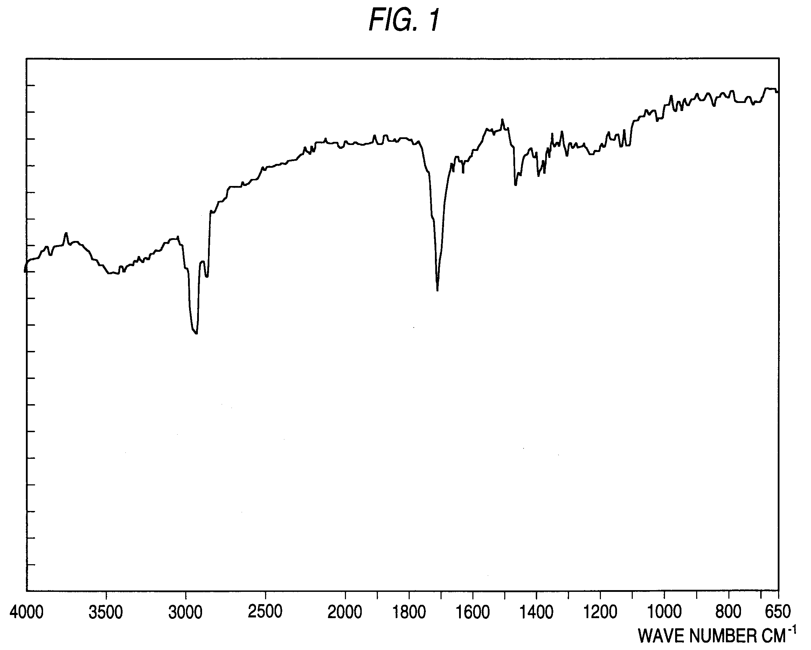

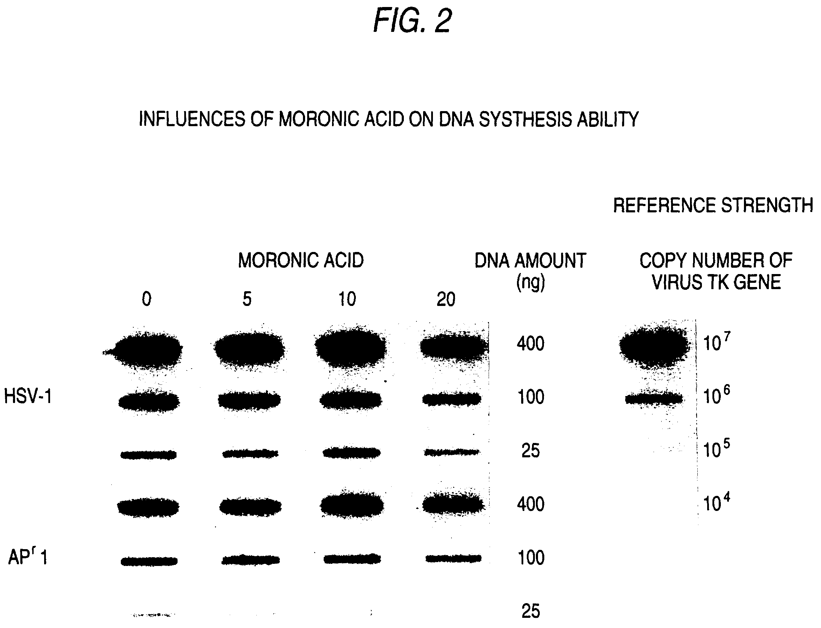

Fig. 1 shows an infrared spectrum of 3-oxo-olean-18-en-28-oic acid. Fig. 2 is a chart showing DNA synthesis inhibition actions of herpesvirus HSV-1 and AP^{r}1 in animal cells examined using a radiation-labeled probe.

### BEST MODE FOR CARRYING OUT THE INVENTION

The examples of the present invention are shown below.

A part of a plant body, insect galls of *Rhus javanica L.*, was used as the crude drug. All materials used in the experiments were commercially available.

9-(2-Hydroxy-ethoxymethyl)guanine (specifically, Aciclovir (referred to as "ACV" hereinafter) tablets manufactured by The Wellcome Foundation Ltd.) was used as a control reagent for comparing various properties of 3-oxo-olean-18-en-28-oic acid of the present invention.

### Example 1

A 500 g portion of insect galls of *Rhus javanica L*. were extracted with 5.2 l of deionized water or distilled water under reflux, and the extract was adjusted to pH 10 by adding 5 N NaOH. The extract was extracted using 6 l of ethyl acetate and then ethyl acetate was removed to obtain 3.1 g of extraction residue (referred to as "EtOAc" extract hereinafter). By repeating the same step six times, 19.1 g of EtOAc extract was obtained.

The residue after removal of ethyl acetate from the above-described pH 10 ethyl acetate extraction fraction was about 2% of the hot water extraction extract. A 4.85 g portion of the ethyl acetate extraction residue was applied to silica gel chromatography, and eluted with 3 ℓ of ethyl acetate : benzene (2 : 8) (fractions 1 to 5), 700 mℓ of methanol : chloroform (3 : 7) (fraction 6) and 300 mℓ of methanol (fraction 7) in this order, thereby fractionating into 7 fractions. Of the 7 fractions, 3.81 g of the first fraction was again applied to silica gel chromatography, and eluted with 3.8 ℓ of hexane (fraction 1), 500 mℓ of chloroform : hexane (2 : 8) (fraction 2), 1 ℓ of chloroform : hexane (4 : 6) (fractions 3 and 4), 1 ℓ of chloroform : hexane (8 : 2) (fractions 5 and 6), 1 ℓ of chloroform (fraction 7), 1 ℓ of methanol : chloroform (2 : 98) (fraction 8), 1 ℓ of methanol : chloroform (5 : 95) (fraction 9) and 1 ℓ of methanol : chloroform (20 : 80) (fraction 10) in this order, thereby fractionating into 10 fractions. Of the 10 fractions, the fourth to seventh fractions were combined and purified by a normal phase thin-layer chromatography using a developing solvent of ethyl acetate : chloroform (1 : 9), thereby obtaining 700 mg of 3-oxo-olean-18-en-28-oic acid.

Outline of the above fractionation treatment is shown in the following.

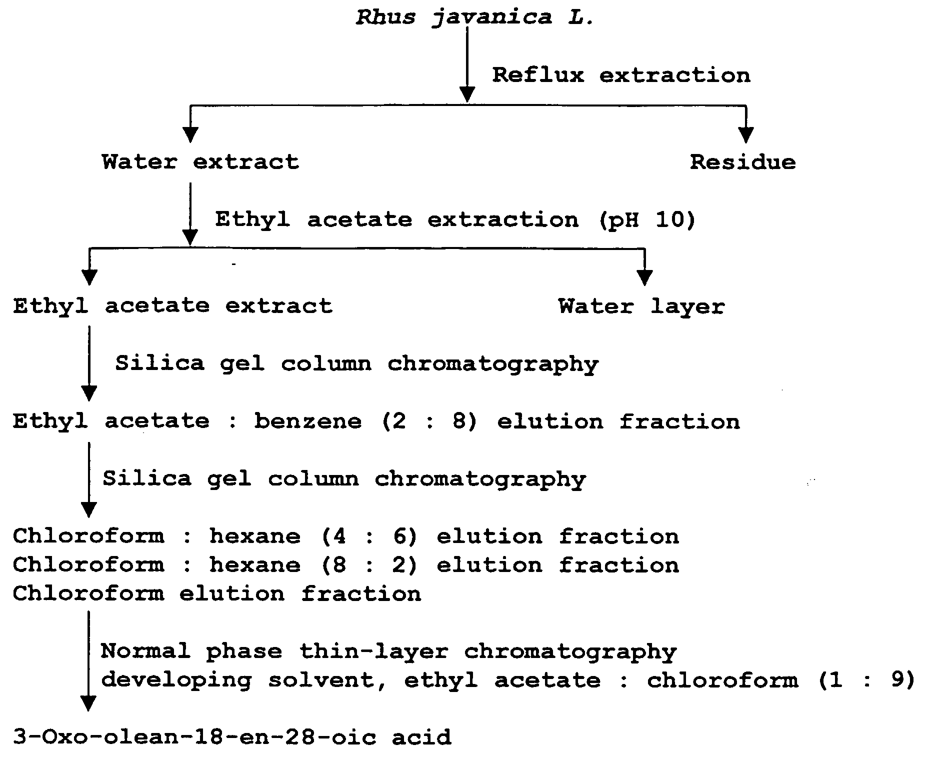

Since infrared spectrum and melting point of this product coincided with the reported values, its structure was confirmed thereby. Infrared spectrum of 3-oxo-olean-18-en-28-oic acid is shown in Fig. 1.

In order to examine antiviral activity, herpes simplex virus type 1 (strain 7401 H) (referred sometimes to as "HSV-1" hereinafter), TK deficiency herpes simplex virus type 1 (strain B 2006) (referred sometimes to as "TK⁻" hereinafter), phosphono acetic acid (referred to as "PAA" hereinafter) resistant herpes simplex virus type 1 (PAA resistant strain derived from 7401 H) (referred sometimes to as "AP^{r}1" hereinafter) and herpes simplex virus type 2 (strain Ito 1262) (referred sometimes to as "HSV-2" hereinafter) were used as viruses.

Vero cells were infected with respective viruses, the thus infected cells after a predetermined period of time were subjected to three times of freezing-thawing and then to centrifugation (3,000 rpm, 15 minutes) and the resulting supernatant fluid was used in the following tests as a virus solution. The virus solution was stored at -80°C. Culturing of the cells was carried out in a carbon dioxide incubator at 37°C in all cases.

Measurement of the antiviral activity was carried out by determining viruses making use of cytopathic effect (CPE) as an index. The determination of viruses was carried out by the following plaque formation test.

That is, the above-described virus solution was diluted to a concentration of 100 plaques/0.2 mℓ, the thus diluted virus solution (0.2 mℓ) was inoculated into Vero cells which have been cultured into monolayer in a plastic dish of 60 mm in diameter, and the mixture was incubated at 37°C for 1 hour to effect adsorption of viruses to the Vero cells. After the adsorption, 2% fetal bovine serum-added MEM medium (5 mℓ) containing each drug solution to be measured having varied drug concentration and 0.8% methyl cellulose was overlaid on the just described virus-adsorbed monolayer Vero cells which were subsequently cultured at 37°C for 2 to 5 days to effect plaque formation. In order to count the number of plaques, the monolayer Vero cells cultured for 2 to 5 days were fixed with formalin and then stained with 0.03% Methylene Blue. The number of plaques was counted under a stereoscopic microscope.

In this connection, the antiviral activity was examined a plurality of times in the above-described manner using each drug solution having the same concentration to confirm that results of the measurement were included within the error range for the confirmatory judgment of plaques.

Table 1 shows the antiviral effect of 3-oxo-olean-18-en-28-oic acid on each virus as 50% plaque formation inhibition concentration (EC₅₀) obtained from the plaque formation test.

The EC₅₀ value of 3-oxo-olean-18-en-28-oic acid, a value which shows 50% plaque formation inhibition concentration of the drug that inhibits 50% of plaque formation by the virus on Vero cells, was 3.9 ± 1.1 µg/mℓ. This value shows that 3-oxo-olean-18-en-28-oic acid (moronic acid) has strong antiviral activity.

**Table 1**

| Antiviral effect of 3-oxo-olean-18-en-28-oic acid (moronic acid) | | | | | |
|---|---|---|---|---|---|
| EC₅₀ (µg/mℓ) (average ± standard deviation) | | | | | |
| Virus tested | | HSV-1 | AP^{r}1 | TK⁻ | HSV-2 |
| Drugs | | | | | |
| ACV | *1 | 0.35 ± 0.05 | >30 | ND *3 | 0.18 ± 0.11 |
| | *2 | 24.4 ± 2.6 | 125.5 ± 12.0 | ND | 14.7 ± 1.04 |
| Moronic acid | | 3.9 ± 1.1 | 1.6 ± 0.4 | 2.0 ± 0.4 | 5.8 ± 0.5 |

| | | | | | |
|---|---|---|---|---|---|
| *1 ACV: trade name Aciclovir | | | | | |
| *2 PAA: phosphono acetic acid | | | | | |
| *3 ND : not detectable | | | | | |

Each of these data was evaluated by the plaque formation test.

Each of the EC₅₀ values was obtained from three experiments on each independent drug. As is evident from Table 1, 3-oxo-olean-18-en-28-oic acid (moronic acid) is effective upon each of the viral strains at almost the same drug level and shows EC₅₀ values at low concentrations particularly against the drug-resistant strains AP^{r}1 and TK⁻. Also, cytotoxicity of 3-oxo-olean-18-en-28-oic acid was not observed at the concentration by which it showed anti-herpesvirus activity.

Also evident from Table 1 is that Aciclovir (ACV) has extremely low effect against the AP^{r}1 strain, as the drug resistance is causing a clinical problem, while the 3-oxo-olean-18-en-28-oic acid (moronic acid) of the present invention can show its effect even against such a drug-resistant strain.

Next, the viral growth inhibition activity was measured in the following manner making use of a plaque forming unit (PFU/mℓ) as an index. That is, each of the above-described viral strains was adsorbed to Vero cells which have been cultured into monolayer in a plastic dish of 60 mm in diameter, and the resulting cells were supplemented with 2% fetal bovine serum-added MEM medium (3 mℓ) and 3-oxo-olean-18-en-28-oic acid solution (1 mℓ) having varied concentration and cultured at 37°C for 24 hours. After 24 hours, the thus infected cells were subjected to three times of freezing-thawing and then to centrifugation (3,000 rpm, 15 minutes), and the resulting supernatant fluid was used as a virus solution to count the number of infectious viruses by the above-described plaque formation test and to calculate the plaque forming unit (PFU/mℓ) based on the counts. The results are shown in Table 2.

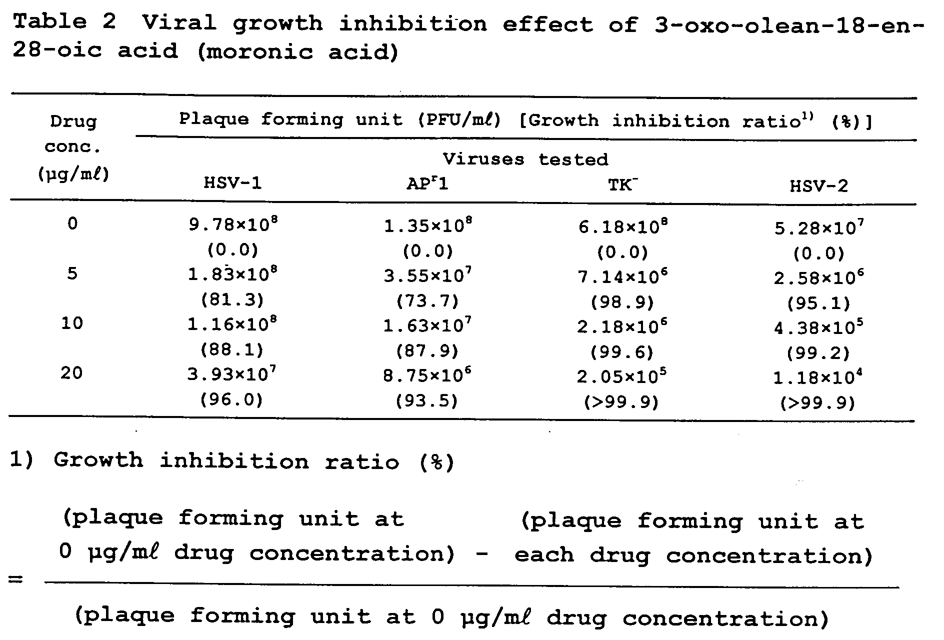

As is evident from Table 2, when each concentration (5, 10 or 20 µg/mℓ) of 3-oxo-olean-18-en-28-oic acid (moronic acid) is compared with the control (water), the ratio of plaques formed by each viral strain decreases as the concentration of 3-oxo-olean-18-en-28-oic acid (moronic acid) increases. This phenomenon can be observed even against the drug-resistant viral strains AP^{r} and TK⁻.

In order to illustratively confirm the effect of 3-oxo-olean-18-en-28-oic acid (moronic acid) to inhibit growth of viruses after viral infection, skin cell tissues of BALB/c mice were infected with the strain HSV-1 and then the parts affected thereafter were observed and scored. The scores were made based on the average time (days ± standard deviation) until the affected parts reached score 2 (local efflorescence, bulla, erosion) or score 6 (slight shingles) and based on the survival days. In order to examine difference in the effects of control and each concentration of 3-oxo-olean-18-en-28-oic acid (moronic acid), 3-oxo-olean-18-en-28-oic acid (moronic acid) was administered to mice three times a day by oral administration (0.25 mℓ per time, the same amount of water as the control) in a dosage of 1 mg/kg in Experiment 1, 0.2 mg/kg, 1 mg/kg or 5 mg/kg in Experiment 2 and 1 mg/kg or 10 mg/kg in Experiment 3, per 1 kg body weight of each mouse, and changes in the morbid states were observed for 14 days after the viral infection.

The results are shown in Table 3.

**Table 3**

| Effects of moronic acid after infection of BALB/c mouse skin tissues with HSV-1 | | | | |
|---|---|---|---|---|
| Exp. No. | Treatment | Average time (days ± standard deviation) | | |
| | | Score-2 (local efflorescence, bulla, erosion) | Score-6 (slight shingles) | Survival |
| Exp.1 | Control (std.) | 3.20 ± 0.42 | 5.10 ± 0.32 | 6.20 ± 0.42 |
| | Moronic acid (1 mg/kg) a | 3.90 ± 0.70 b | 5.70 ± 0.68 b | 7.30 ± 1.16 b |
| Exp.2 | Control (std.) | 3.30 ± 0.48 | 5.33 ± 0.50 | 6.56 ± 0.73 |
| | Moronic acid (0.2 mg/kg) c | 4.00 ± 0.47 d | 5.78 ± 0.97 | 6.89 ± 0.33 |
| | Moronic acid (1 mg/kg) | 3.60 ± 0.70 | 5.40 ± 0.52 | 7.00 ± 1.49 |
| | Moronic acid (5 mg/kg) | 3.90 ± 0.74 b | 5.40 ± 0.53 | 7.10 ± 1.52 |
| Exp. 3 | Control (std.) | 3.20 ± 0.42 | 5.20 ± 0.42 | 6.60 ± 0.52 |
| | Moronic acid (1 mg/kg) (ANOVA: 0.056 repeated measurement) | 3.80 ± 0.63 b | 5.60 ± 0.71 | 7.70 ± 2.16 |
| | Moronic acid (10 mg/kg) | 3.70 ± 0.48 b | 5.30 ± 0.48 | 6.80 ± 0.42 |

| | | | | |
|---|---|---|---|---|
| a: Indicates that there is a significant difference with remeasurement of the control (standard) having a level of significance (P) of 0.01 or less by ANOVA (analysis of valiance). | | | | |
| b: Indicates that there is a significant difference the control (standard) having a level of significance (P) of 0.05 or less by Student's t-test. | | | | |
| c: Indicates that there is a significant difference with remeasurement of the control (standard) having a level of significance (P) of 0.05 or less by ANOVA (analysis of valiance). | | | | |
| d: Indicates that there is a significant difference the control (standard) having a level of significance (P) of 0.01 or less by Student's t-test. | | | | |

The above-described level of significance (P) and t-test are described. The t-test is a method in which, when there are two populations, whether or not there is a significant difference between these two populations, namely whether or not these populations can be regarded as the same population, is tested (judged) by a statistical means, and the probability that the results of the t-test is an error is expressed as a level of significance (P). When the level of significance (P) is 0.01 or less, it means that the probability of the results of the t-test being an error is 0.01 or less, namely 1% or less.

In this connection, the symbol "a" in Table 3 means the result of comparison when the control and 1 mg/kg of moronic acid were used, each of the symbols "b" and "d" means the result of comparison with the value of respective control shown just above each symbol and the symbol "c" means the result of comparison when the control and 0.2 mg/kg of moronic acid were used.

As is evident from Table 3, when differences between the control and each concentration of 3-oxo-olean-18-en-28-oic acid (moronic acid) are compared in terms of the average days when reached the score 2 and score 6, as well as of the survival days, a distinctive degree of significant difference which indicates delaying of the generation of viruses can partly be seen in the 3-oxo-olean-18-en-28-oic acid (moronic acid) administered groups in all of the experiments 1, 2 and 3. In describing the measured data marked with the symbols a, b, c and d, each of a and c indicates a safety meaning re-measurement by ANOVA (analysis of valiance), and b and d indicate data which have a significant difference in moronic acid-treated groups, obtained by calculating the probability of the level of significance (P) by Student's t-test.

Accordingly, the data marked with the symbols a and d have a level of significance (P) of 1% or less, and those marked with b and c have a level of 5% or less. On the basis of these results, it was confirmed that 3-oxo-olean-18-en-28-oic acid (moronic acid) has an effect in treating herpesvirus infections.

Next, effects of 3-oxo-olean-18-en-28-oic acid (moronic acid) against a virus in the animal body were examined by infecting skin cell tissues of mice with the viral strain HSV-1 and measuring the amount of the virus particles remained in the affected part of the skin and those migrated from the skin into intracerebral nerve cells 2, 3, 4 and 6 days after the infection, when a control (water only) or a varied amount of 3-oxo-olean-18-en-28-oic acid (moronic acid) was administered to the mice. In each of the experiments 1 and 2, three mice were used in one group and the average number of virus particles obtained from each mouse was used as the amount of virus. The amount of virus was measured by cutting off an area of 1 cm² around the affected part in the case of the skin or using the entire brain portion in the case of the brain. Each of the tissue samples was mixed with 2 mℓ of physiological saline and homogenated, the supernatant fluid obtained therefrom was used as a virus test solution to carry out a plaque assay (plaque measuring method) using cultured Vero cells, and the number of plaques thus counted was used as the amount of virus. The skin/brain ratio is a value calculated as the ratio of respective percentages obtained from the skin and the brain.

The results are shown in Tables 4 and 5.

With regard to the numeral in ( ) such as (100%), (65.4%) or the like shown in the item of the amount of virus, the amount of virus in the control was defined as 100% and shown as (100%), the ratio of residual virus particles when 10 mg/kg of moronic acid was administered was 65.4% so that it was shown as (65.4%), and other numerals in ( ) under respective data were shown in the same manner.

**Table 4**

| Effects of 3-oxo-olean-18-en-28-oic acid (moronic acid) on the amount of virus in the brain and skin of mice infected with HSV-1 | | | | |
|---|---|---|---|---|
| Days after infection | Treatment | Amount of virus (average ± standard deviation, PFU/organ) | | Skin/brain (ratio) |
| | | Skin (log₁₀ PFU)/cm² | Brain (log₁₀ PFU)/total | |
| Exp. 1 | | | | |
| 2 days | control | 5.3 ± 0.8 (100%) | ND | |
| | moronic acid (10 mg/kg) | 4.9 ± 0.0 (91.2) | ND | |
| 4 days | control | 6.6 ± 0.1 (100%) | 2.9 ± 0.2 (100%) | 1 |
| | moronic acid (1 mg/kg) | 6.1 ± 0.4 (93.5%) | <2.4 ± 0.0 <(83.9%) | >1.12 |
| | moronic acid (10 mg/kg) | 6.3 ± 0.3 (96.3%) | <2.40 ± 0.0 <(83.9%) | >1.15 |
| 6 days | control | 6.4 ± 0.3 (100%) | 4.1 ± 0.3 (100%) | 1 |
| | moronic acid (1 mg/kg) | 6.1 ± 0.2 (95.3%) | 3.5 ± 0.4 (85.6%) | 1.12 |
| | moronic acid (10 mg/kg) | 6.0 ± 0.1 (94.2%) | 2.3 ± 0.3 (57.0) | 1.674 |
| ND: not detectable | | | | |

**Table 5**

| Effects of 3-oxo-olean-18-en-28-oic acid (moronic acid) on the amount of virus in the brain and skin of mice infected with HSV-1 | | | | |
|---|---|---|---|---|
| Days after infection | Treatment | Amount of virus (average ± standard deviation, PFU/organ) | | Skin/brain (ratio) |
| | | Skin (log₁₀ PFU)/cm² | Brain (log₁₀ PFU)/total | |
| Exp. 2 | | | | |
| 3 days | control | 5.6 ± 0.1 (100%) | ND | |
| | moronic acid (1 mg/kg) | 5.5 ± 0.4 (98.2%) | ND | |
| | moronic acid (5 mg/kg) | 5.1 ± 0.4 (91.5%) | ND | |
| 6 days | control | 5.1 ± 0.3 (100%) | 3.7 ± 1.2 (100%) | 1 |
| | moronic acid (1 mg/kg) | 4.8 ± 0.7 (93.9%) | 3.0 ± 0.6 (80.7%) | 1.16 |
| | moronic acid (5 mg/kg) | 5.6 ± 0.2 (109.0%) | 2.7 ± 0.3 (72.2%) | 31.9 |
| ND: not detectable | | | | |

As can be seen in Tables 4 and 5, in comparison with the control, the number of virus particles is reduced by the treatment with 3-oxo-olean-18-en-28-oic acid (moronic acid) almost dependently on the concentration of 3-oxo-olean-18-en-28-oic acid (moronic acid), both in the intracerebral tissue and the skin tissue in all cases after 2, 3, 4 and 6 days of the infection, so that it is evident that not only this compound inhibits growth of the virus in the skin of the affected part but also it particularly and markedly inhibits amount of the virus to be migrated into the brain. Thus, since it can reduce migration of the herpesvirus into the nervous system after infection and inhibit the viral growth, its effect against palindromic crisis is suggested. In other words, it was confirmed that moronic acid has an effect to prevent recurrence of herpesvirus infections.

Next, in order to examine toxicity of moronic acid, a toxicity test was carried out by administering control (water only) or 1 mg, 5 mg or 10 mg per kg mouse body weight of 3-oxo-olean-18-en-28-oic acid (moronic acid) to mice which have not been infected with viruses. Mice having a weight of 18 to 21 g/animal just before the commencement of the test were used in five animals per group. None of the mice died before completion of the test. 3-Oxo-olean-18-en-28-oic acid (moronic acid) was orally administered every day and three times a day.

The results are shown in Table 6.

**Table 6**

| Toxicity of 3-oxo-olean-18-en-28-oic acid (moronic acid) upon non-infected mice | | | | |
|---|---|---|---|---|
| Treatment | (Average body weight ± standard deviation) | | | Death rate |
| | Administration period | | | |
| | 7 days | 21 days | 36 days | |
| Exp. 1 | | | | |
| Control | 19.5 ± 1.1 | not tested | 22.2 ± 1.2 | 0/5 |
| Moronic acid (1 mg/kg) | 19.8 ± 1.4 | not tested | 22.6 ± 0.8 | 0/5 |
| Moronic acid (5 mg/kg) | 19.9 ± 1.4 | not tested | 21.0 ± 0.8 | 0/5 |

| Exp. 2 | | | | |
|---|---|---|---|---|
| Control | 20.2 ± 0.8 | 20.8 ± 0.8 | not tested | 0/5 |
| Moronic acid (10 mg/kg) | 20.1 ± 1.2 | 20.4 ± 1.0 | not tested | 0/5 |

As shown in Table 6, body weights of five mice hardly changed with the lapse of days when the control or 3-oxo-olean-18-en-28-oic acid (moronic acid), even at the maximum concentration of 10 mg/kg, was administered, and none of the five mice died after the lapse of time of 25 days. These results confirm that moronic acid exerts almost no toxicity upon animal cells.

Finally, in order to examine effects of moronic acid on the viral synthesis ability, the amount of virus particles in cultured cells was measured by the slot-blot hybridization described in *J. Traditional Med*., 12, 187 - 194 (1995). Using a radiation-labeled probe, viral DNA synthesis ability of herpesvirus strains HSV-1 and AP^{r}1 was measured just after adsorption of the viruses to animal cells (Vero cells) or when concentration of 3-oxo-olean-18-en-28-oic acid (moronic acid) was changed (0, 5, 10 or 20 µg/mℓ), with the results shown as a chart in Fig. 2 (photograph).

In Fig. 2, rough amounts of each herpesvirus are shown by the strength of black parts. For the sake of comparison, DNA copy number of a virus TK⁻ is shown on the right-side column as a reference strength.

As shown in Fig. 2, effect of 3-oxo-olean-18-en-28-oic acid (moronic acid) as a drug to inhibit virus DNA synthesis was not found even at an increased concentration of 20 µg/mℓ, so that it was confirmed that the anti-viral effect of moronic acid is not due to its DNA synthesis inhibition but based on its effect to reduce the number of virus particles.

### INDUSTRIAL APPLICABILITY

As is evident from the test results shown in Tables 1 to 6, 3-oxo-olean-18-en-28-oic acid (moronic acid) has an effect to inhibit growth of viruses and, since it also inhibits viruses migrating into the nervous system, palindromic crisis of herpesvirus in latent stage in the nervous system is inhibited, thus confirming that it has a significant recurrence preventing effect in addition to its effect to treat herpesvirus infections and that it hardly causes side effects.

In addition, it is fully possible to use the 3-oxo-olean-18-en-28-oic acid of the present invention together with Aciclovir.

## Claims

1. An agent for treating herpesvirus infections, which comprises 3-oxo-olean-18-en-28-oic acid represented by the following formula (I): 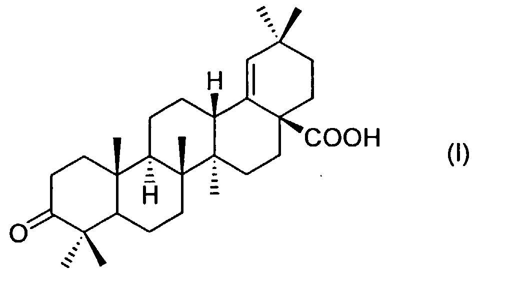 as an active ingredient.

2. An agent for preventing recurrence of herpesvirus infections, which comprises 3-oxo-olean-18-en-28-oic acid represented by the following formula (I): 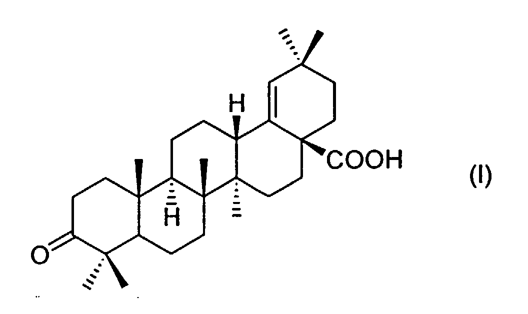 as an active ingredient.
